# EUROPEAN PATENT SPECIFICATION

(11) **EP 0 285 057 B1**
(45) Date of publication and mention of the grant of the patent: **01.03.1995**
(21) Application number: 88104961.3
(22) Date of filing: 22.06.1983
(51) Int. Cl.: C07H 21/00, C07H 19/073, C07H 19/10, C07H 19/14, G01N 33/58

(54) **Modified labeled nucleotides and polynucleotides and methods of preparing, utilizing and detecting same**
Markierte modifizierte Nukleotide und Polynukleotide und Verfahren zu deren Herstellung, Verwendung und Detektion
Nucléotides et polynucléotides modifiés marqués et méthodes pour leur préparation, utilisation et détection

(30) Priority: 23.06.1982 US 391440
(43) Date of publication of application: 05.10.1988
(62) Divisional of application: 83106112.2
(73) Proprietor: ENZO BIOCHEM, INC., New York, N.Y. 10013 (US)
(72) Inventor: Engelhardt, Dean, New York, N.Y. 10024 (US); Rabbani, Elazar, New York, N.Y. 10003 (US); Kline, Stanley, Brooklyn, N.Y. 11217 (US); Stavrianopoulos, Jannis G., New York, N.Y. 10019 (US); Kirtikar, Dollie, Elmhurst, N.Y. 11373 (US)
(74) Representative: VOSSIUS & PARTNER

(56) References cited:
- US-A- 3 960 840
- US-A- 4 255 566
- CHEMICAL ABSTRACTS, vol. 94, no. 25, 22nd June 1981, page 366, abstract no.214298t, Columbus, Ohio, US; P. CLECHET et al.: & ANALYSIS 1981, 9(4), 125-8
- CHEMICAL ABSTRACTS, vol. 66, no. 9, 27th February 1967, page 3326, abstract no.35045h, Columbus, Ohio, US; J.A. DUKE et al.: "Conformational changeaccompanying modification of myosin ATPase", & BIOCHIM. BIOPHYS. ACTA126(3), 600-3

## Description

It is known to produce nucleotides or polynucleotides which are radioactively labeled, such as with isotopes or hydrogen (³H), phosphorus (³²P), carbon (¹⁴C) or iodine (¹²⁵I). Such radioactively labeled compounds are useful to detect, monitor, localize and isolate nucleic acids and other molecules of scientific or clinical interest. Unfortunately, however, the use of radioactively labeled materials presents hazards due to radiation. Also due to the relatively short half life of the radioactive materials employed to label such compounds or materials, the resulting labeled compounds or materials have a corresponding relatively short shelf life.

It has been proposed to chemically label compounds of interest, such as nucleotides and polynucleotides, so as to overcome or avoid the hazards and difficulties associated with such compounds or materials when radioactively labeled. In the article by P.R. Langer, A. A. Waldrop and D. C. Ward entitled "Enzymatic Synthesis of Biotin-Labeled Polynucleotides: Novel Nucleic Acid Affinity Probes", in Proc. Natl. Acad. Sci., USA, Vol. 78, No. 11, pp. 6633-6637, November, 1981, there are described analogs of dUTP and UTP that contain a biotin molecule bound to the C-5 position of the pyrimidine ring through an alkylamine linker arm. The biotin-labeled nucleotides are efficient substrates for a variety of DNA and RNA polymerases in vitro. Polynucleotides containing low levels of biotin substitution (50 molecules or fewer per kilobase) have denaturation, reassociation and hybridisation characteristics similar to those of unsubstituted controls. Biotin-labeled polynucleotides, both single and double stranded, are selectively and quantatively retained on avidin-Sepharose, even after extensive washing with 8M urea, 6M guanidine hydrochloride or 99% formamide. In addition, biotin-labeled nucleotides can be selectively immunoprecipitated in the presence of antibiotin antibody and Staphylococcus aureus, Protein A. These unique features of biotin-labeled polynucleotides suggest that they are useful affinity probes for the detection and isolation of specific DNA and RNA sequences.

The subject matter of said article is comprised in EP-A2-0063879 in which additionally it is disclosed that compounds having the structure: 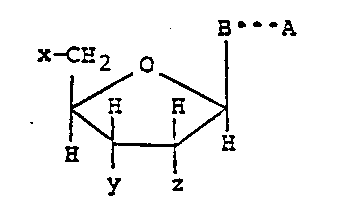
wherein B represents a purine, deazapurine, or pyrimidine moiety covalently bonded to the C^{1'}-position of the sugar moiety, provided that when B is purine or 7-deazapurine, it is attached at the N⁹-position of the purine or deazapurine, and when B is pyrimidine, it is attached at the N¹-position;
wherein A represents a moiety consisting of at least three carbon atoms which is capable of forming a detectable complex with a polypeptide when the compound is incorporated into a double-stranded ribonucleic acid, deoxyribonucleic acid duplex, or DNA-RNA hybrid;
wherein the dotted line represents a chemical linkage joining B and A, provided that if B is purine the linkage is attached to the 8-position of the purine, if B is 7-deazapurine, the linkage is attached to the 7-position of the deazapurine, and if B is pyrimidine, the linkage is attached to the 5-position of the pyrimidine; and
wherein each of x, y,and z represents 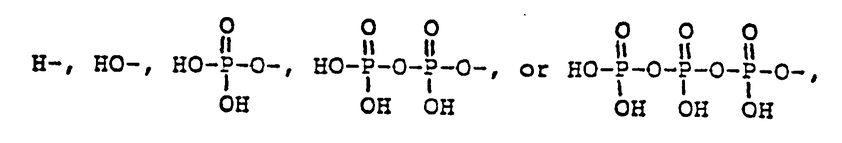
either directly, or when incorporated into oligo- and polynucleotides, provide probes which are widely usely.

Applications disclosed in EP-A2-0 063 879 include detection and localization of polynucleotide sequences in chromosomes, fixed cells, tissue sections and cell extracts. Specific applications include chromosomal karyotyping, clinical diagnosis of nucleic acid-containing etiological agents, e.g. bacteria, viruses, or fungi, and diagnosis of genetic disorders.

By way of additional background with respect to the utilization of the biotin-polynucleotides of the above-identified Langer et al article in Proc. Natl. Acad. Sci. USA, the publication by P. R. Langer-Safer, M. Levine and D. C. Ward in Genetics entitled "An Immunological Method for Mapping Genes on Drosophila Polytene Chromosomes", describes a method employing biotinated nucleotides as a probe for the localization of DNA sequences hybridized in situ to Drosophila polytene chromosomes. In this application these probes are detected using affinity purified rabbit antibiotin antibody as the primary antibody and fluoresceinated goat antirabbit antibody as the secondary antibody.

Other techniques employing biotin-labeled reagents with avidin or enzyme-labeled avidin reagents are known for the detection and determination of ligands in a liquid medium, see U.S. Patent 4,228,237. Also, it is known to effect gene enrichment based on avidin-biotin interaction, particularly as applied to Drosophila ribosomal RNA genes, see the J. Manning, M. Pellegrini and N. Davidson publication in Biochemistry, Vol. 16, No. 7, pages 1364-1369 (1977). Other publications of background interest with respect to the practices of this invention are the D. J. Eckermann and R. H. Symons article entitled "Sequence at the Site of Attachment of an Affinity-Label Derivative of Puromycin on 23-S Ribosomal RNA of Escherichia coli Ribosomes", J. Biochem, 82, 225-234 (1978); the article by S. B. Zimmerman, S. R. Kornberg and A. Kornberg entitled "Glucosylation of Deoxyribonucleic Acid-II -Glucosyl Transferases from T2- and T6-Infected Escherichia coli in The Journal of Biological Chemistry, Vol. 237, No. 2, February 1962, and the article by J. Josse and A. Kornberg "III. α-and β-Glucosyl Transferases from T4-Infected Escherichia coli", also appearing in The Journal of Biological Chemistry, Vol. 237, No. 6, June 1962.

Of further interest in connection with the practices of this invention are the publications appearing in the J. Biol. Chem., Vol. 236, No. 5, May 1961, pages 1487-1493; the same publication, Vol. 237, No. 4, pages 1251-1259 (1962); the same publication Vol. 239, No. 9, pages 2957-2963 (1964). Of special interest is the article appearing in The Journal of Histochemistry and Cytochemistry, Vol. 27, No. 8, pages 1131-1139 (1979) and in the publication Nucleic Acids Research, Vol. 5, No. 9, 1977, pages 2961-2973. Also of interest is the article appearing in the publication Biochimica et Biophysica Acta by A. De Waard entitled "Specificity Difference Between the Hydroxymethylcytosine β-Glucosyl-Transferases Induced by Bacteriophages T2, T4 and T6", pages 286-304, and also the article by T. W. North and C. K. Mathews entitled "T4 Phage-Coded Deoxycytidylate Hydroxymethylase: Purification and Studies in Intermolecular Interactions", published by Academic Press, 1977, pages 898-904 and the article by E. A. Bayer and M. Wilchek entitled "The Use of Avidin-Biotin Complex as a Tool in Molecular Biology in Methods of Biochemical Analysis, Vol. 26, pages 1-45 (1980).

Other techniques useful in the practices of this invention include nick translation of DNA employing DNA polymerase. A technique for effecting nick translation is disclosed in the article by P. W. Rigby, M. Dieckmann, C. Rhodes and P. Berg entitled "Labeling Deoxyribonucleic Acid to High Specific Activity in vitro by Nick Translation with DNA Polymerase" in J. Mol. Biol. (1977), 113, 237-251. With respect to the recovery of streptavidin, such as from a culture broth of Streptomyces avidinii, the article by K. Hofmann, S. W. Wood, C. C. Brinton, J. A. Montibeller and F. M. Finn entitled "Iminobiotin Affinity Columns and their Application to Retrieval of Streptavidin" in Proc. Natl. Acad. Sci. USA, Vol. 77, No. 8, pp. 4666-4668 (1980), discloses a suitable approach for the recovery of streptavidin from a strepavidin-containing material, such as from a culture broth. Streptavidin is useful as a reagent in one of the practices of this invention.

The present invention relates to nucleotides which are modified, such as at the 5 position of pyrimidine or the 7 position of purine, preparatory for the preparation therefrom of nucleotide probes suitable for attachment to or incorporation into DNA or other nucleic acid material. In the practices of this invention nucleotides, i.e. nucleic acids, preferably are modified in a non-disruptive manner such that the resulting modified nucleotides are capable of incorporation into nucleic acids and once incorporated in nucleic acids the modified nucleotides do not significantly interfere with the formation or stabilization of the double helix formed of the resulting nucleic acids containing the modified nucleotides. The non-disruptive modification of nucleotides and nucleic acids incorporating such modified nucleotides is in contrast with those modifications of nucleotides which are characterized as a disruptive modification in the sense that the resulting disruptively modified nucleotides and nucleic acids containing the same block proper double helix formation. In the practices of this invention, the nucleotides are desirably modified at the 5 position of the pyrimidine or the 7 position of the purine. The nucleotides so modified are non-disruptively modified and nucleic acids containing such nucleotides are capable of forming a double helix arrangement. In the practices of this invention, the nucleotides are also modified in the disruptive positions, as set forth in the description that now follows.

The present invention relates to a nucleotide to which is covalently attached a moiety that is capable of chelating a ion, whereby the nucleotide is selected from the group consisting of:
(i) a nucleotide having the formula

   P-S-B-Sig

   wherein P is a phosphate moiety, S is a sugar moiety, B is a pyrimidine, purine or 7-deazapurine moiety, Sig Comprises a moiety capable of chelating a detectable ion, P being attached at the 3' or the 5' position of S when the nucleotide is a deoxyribonucleotide and at the 2', 3' or 5' position when the nucleotide is a ribonucleotide, B being attached to the 1' position of S from the N¹ position when B is a pyrimidine or the N⁹ position when B is a purine or a 7-deazapurine, and Sig is directly or indirectly attached to B at a position other than the C⁵ position when B is a pyrimidine, at a position other than the C⁸ position when B is a purine and at a position other than the C⁷ position when B is a 7-deazapurine;
(ii) a ribonucleotide having the formula, 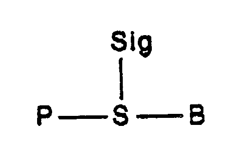 wherein P is a phosphate moiety, S is a sugar moiety, B is a pyrimidine, purine or 7-deazapurine moiety, and Sig comprises a moiety capable of chelating a detectable ion, P being attached at the 2', 2' or 5' position of S, B being attached to the 1' position of S from the N¹ position when B is a pyrimidine or the N⁹ position when B is a purine or 7-deazapurine; and Sig is directly or indirectly attached to S; and
(iii) a nucleotide having the formula, 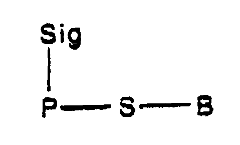 wherein P is a phosphate moiety, S is a sugar moiety, B is a pyrimidine purine or 7-deazapurine moiety, Sig comprises a moiety capable of chelating a detectable ion, P being attached to the 3' or the 5' position of S when said nucleotide is a deoxyribonucleotide and at the 2', 3' or 5' position when said nucleotide is a ribonucleotide, B being attached to the 1' position of S from the N¹ position when B is a pyrimidine or the N⁹ position when B is a purine, and wherein Sig is directly or indirectly attached to P.

Preferred embodiments of the present invention are explained in detail in the following enumeration.
1. A nucleotide having the general formula P-S-B-Sig wherein P is the phosphoric acid moiety, S the sugar or monosaccharide moiety, B being the base moiety, the phosphoric acid moiety being attached at the 3' and/or the 5' position of the sugar moiety when said nucleotide is a deoxyribonucleotide and at the 2', 3' and/or 5' position when said nucleotide is a ribonucleotide, said base being a purine or a pyrimidine, said base being attached from the N1 position or the N9 position to the 1' position of the sugar moiety when said base is a pyrimidine or a purine, respectively, and wherein said Sig comprises a moiety capable of chelating a detectable ion.
2. A nucleotide in accordance with item 1 wherein said nucleotide is a deoxyribonucleotide.
3. A nucleotide in accordance with item 1 wherein said nucleotide is a ribonucleotide.
4. A nucleotide in accordance with item 1 wherein said chemical moiety Sig is chemically attached to B at the N7 position when B is a 7-deazapurine, at the C5 position when B is a pyrimidine and at the C8 position when B is a purine.
5. A nucleotide in accordance with item 1 wherein Sig is attached to B at a position such that an oligonucleotide or polynucleotide containing said nucleotide is capable of forming a double-stranded ribonucleic acid, a double-stranded deoxyribonucleic acid or a DNA-RNA hybrid, or when said nucleotide is incorporated into said oligonucleotide or polynucleotide.
6. A nucleotide in accordance with item 1 wherein Sig is attached to B at a position such that said nucleotide is capable of being incorporated into or to form a double-stranded ribonucleic acid, a double-stranded deoxyribonucleic acid or a double-stranded deoxyribonucleic acid-ribonucleic acid hybrid.
7. A nucleotide in accordance with item 1 wherein Sig is attached to B at a position such that when said nucleotide is incorporated into or attached to or associated with a double-stranded deoxyribonucleic acid or double-stranded ribonucleic acid or DNA-RNA hybrid, said chemical moiety Sig is capable of signalling itself or making itself self-detecting or its presence known.
8. An oligonucleotide or polydeoxyribonucleotide comprising one or more nucleotides in accordance with item 1.
9. An oligonucleotide or polyribonucleotide comprising one or more nucleotides in accordance with item 1.
10. A nucleotide in accordance with item 1 wherein said Sig is attached to said base B at the C5 position when said base B is a pyrimidine or at the C7 position when said base B is a deazapurin.
11. A nucleotide in accordance with item 1 wherein said base B is a pyrimidine and wherein said Sig chemical moiety is attached to the pyrimidine at the N3 position.
12. A nucleotide in accordance with item 1 wherein said base B is a pyrimidine and wherein said Sig chemical moiety is attached to the pyrimidine at the C5 position.
13. A nucleotide in accordance with item 1 wherein said base B is a pyrimidine and wherein said Sig chemical moiety is attached to the pyrimidine at the C6 position.
14. A nucleotide in accordance with item 1 wherein said base B is a purine and wherein said Sig chemical moiety is attached to the purine at the N1 position.
15. A nucleotide in accordance with item 1 wherein said base B is a purine and wherein said Sig chemical moiety is attached to the purine at the C2 position.
16. A nucleotide in accordance with item 1 wherein said base B is a purine and wherein said Sig chemical moiety is attached to the purine at the N3 position.
17. A nucleotide in accordance with item 1 wherein said base B is a purine and wherein said Sig chemical moiety is attached to the purine at the N7 position.
18. A nucleotide in accordance with item 1 wherein said base B is a purine and wherein said Sig chemical moiety is attached to the purine at the C8 position.
19. A nucleotide in accordance with item 1 wherein said S sugar is a pentose.
20. A nucleotide in accordance with item 1 wherein said base B is a 7-deazapurine and wherein said Sig chemical moiety is attached to the 7-deazapurine at the C7 position.
21. A nucleotide in accordance with item 1 wherein said Sig chemical moiety includes or comprises a radioactive isotope.
22. A nucleotide in accordance with item 21 wherein said radioactive isotope is radioactive cobalt.
23. A Single-stranded polynucleotide comprising one or more nucleotides in accordance with item 1.
24. A single-stranded polynucleotide in accordance with item 23 wherein said single-stranded polynucleotide is a polydeoxyribonucleotide.
25. A single-stranded polynucleotide in accordance with item 23 wherein said single-stranded polynucleotide is a polyribonucleotide.
26. A double-stranded polynucleotide comprising one or more nucleotides in accordance with item 1.
27. A double-stranded polynucleotide in accordance with item 26 wherein said double-stranded polynucleotide is a double-stranded deoxyribonucleic acid.
28. A double-stranded polynucleotide in accordance with item 26 wherein said double-stranded polynucleotide is a double-stranded ribonucleic acid.
29. A double-stranded polynucleotide in accordance with item 26 wherein said double-stranded polynucleotide is a double-stranded deoxyribonucleic acid-ribonucleic acid hybrid.
30. A single-stranded polynucleotide comprising at least 12 nucleotides, at least one of said nucleotides being a nucleotide in accordance with item 1.
31. A nucleotide in accordance with item 1 wherein said base B is cytosine.
32. A nucleotide in accordance with item 1 wherein said base B is uracil.
33. A nucleotide in accordance with item 1 wherein said base B is thymine.
34. A nucleotide in accordance with item 1 wherein said base B is adenine.
35. A nucleotide in accordance with item 1 wherein said base B is guanine.
36. A nucleotide in accordance with item 1 wherein said base B is 2-methyladenine.
37. A nucleotide in accordance with item 1 wherein said base B is 1-methylguanine.
38. A nucleotide in accordance with item 1 wherein said base B is 5-methylcytosine.
39. A nucleotide in accordance with item 1 wherein said base B is 5-hydroxymethylcytosine.
40. A nucleotide in accordance with item 1 wherein said Sig chemical moiety comprises a chelating agent.
41. A nucleotide in accordance with item 1 wherein said base B is deazaadenine.
42. A nucleotide in accordance with item 1 wherein said base B is deazaguanine.
43. A nucleotide in accordance with item 1 wherein said Sig chemical moiety is connected to said base B via a chemical linkage.
44. A nucleotide in accordance with item 43 wherein said chemical linkage includes an olefinic bond at the a-position relative to base B.
45. A nucleotide in accordance with item 43 wherein said chemical linkage includes the moiety,

   - CH₂ - NH - .
46. A nucleotide in accordance with item 43 wherein said chemical linkage is,

   - CH = CH - CH₂ - NH - .
47. A nucleotide in accordance with item 1 wherein the chemical moiety is, 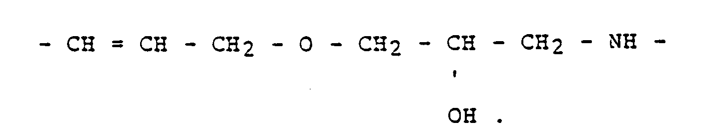
48. A nucleotide in accordance with item 46 wherein said Sig chemical moiety is attached to the - NH - group of said chemical linkage.
49. A ribonucleotide having the general formula, 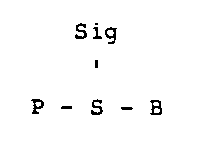 wherein P is a phosphate moiety, S is a sugar moiety, B is a pyrimidine, purine or 7-deazapurine moiety, and Sig comprises a moiety capable of chelating a detectable ion, P being attached at the 2', 3' or 5' position of S, B being attached to the 1' position of S from the N¹ position when B is a pyrimidine or the N⁹ position when B is a purine or 7-deazapurine; and Sig is directly or indirectly attached to S.
50. A ribonucleotide in accordance with item 49 wherein said Sig chemical moiety is attached to the C2' position of said sugar moiety.
51. A ribonucleotide in accordance with item 49 wherein said Sig chemical moiety is attached to the C3' position of said sugar moiety.
52. A ribonucleotide in accordance with item 49 wherein said Sig chemical moiety is attached to the S sugar moiety such that an oligoribonucleotide or polyribonucleotide containing said ribonucleotide is capable of forming a double-stranded ribonucleic acid or a DNA-RNA hybrid when said ribonucleotide is incorporated into said oligoribonucleotide or said polyribonucleotide.
53. A polyribonucleotide comprising at least one ribonucleotide in accordance with item 49.
54. A ribonucleotide in accordance with item 1 wherein said base B is a pyrimidine.
55. A ribonucleotide in accordance with item 49 wherein said base B is a purine.
56. A ribonucleotide in accordance with item 49 wherein said base B is uracil.
57. A ribonucleotide in accordance with item 49 wherein said base B is adenine.
58. A ribonucleotide in accordance with item 49 wherein said base B is guanine.
59. A ribonucleotide In accordance with item 49 wherein said base B is cytosine.
60. A ribonucleotide in accordance with item 49 wherein said base B is a 7-deazapurine.
61. A ribonucleotide in accordance with item 49 wherein said Sig chemical moiety includes or comprises a radioactive isotope component.
62. A ribonucleotide in accordance with item 61 wherein said radioactive isotope component is radioactive cobalt.
63. A single stranded polyribonucleotide comprising one or more ribonucleotides in accordance with item 49, said single stranded polyribonucleotide comprising at least three said ribonculeotides.
64. A single stranded polyribonucleotide comprising at least twelve ribonucleotides and containing at least one ribonucleotide in accordance with item 49.
65. A nucleotide having the general formula 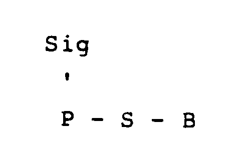 wherein P is a phosphate moiety, S is a sugar moiety, B is a pyrimidine, purine or 7-deazapurine moiety, Sig comprises a moiety capable of chelating a detectable ion, P being attached to the 3' or the 5' position of S when odd nucleotide is a deoxyribonucleotide and at the 2', 3' or 5' position when said nucleotide is a ribonucleotide, B being attached to the 1' position of S from the N¹ position when B is a pyrimidine or the N⁹ position when B Is a purine, and wherein Sig is directly or indirectly attached to P.
66. A nucleotide in accordance with any of the previous items wherein said chelating agent includes the chemical moiety 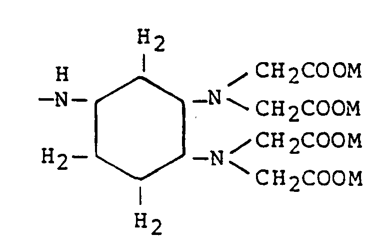 wherein M is H or a substitutable metal.
67. A nucleotide in accordance with item 66 wherein said metal is magnesium or a metal replaceable by cobalt.
68. A polynucleotide containing one or more nucleotides in accordance with item 1 or item 49 or item 65 wherein said Sig chemical moiety includes a chelating agent.
69. A polynucleotide containing a nucleotide in accordance with item 1 or item 49 or item 65 wherein said Sig chemical moiety includes a chelating agent in accordance with Claim 66.
70. A nucleotide in accordance with item 66 wherein said metal is a catalytically active metal.
71. A nucleotide in accordance with item 66 wherein said metal is a heavy metal.
72. A nucleotide in accordance with item 66 wherein said metal is radioactive cobalt.
73. A nucleotide in accordance with item 66 wherein said metal is magnesium.
74. A ribonucleotide in accordance with item 1 or item 49 or item 65 wherein said Sig chemical moiety includes a chelating agent.
75. A deoxyribonucleotide in accordance with item 1 or item 49 item 65 wherein said Sig chemical moiety includes a chelating agent.
76. A nucleotide in accordance with item 66 wherein said metal M is a radioactive isotope.
77. A nucleotide in accordance with item 66 wherein said metal is platinum.
78. A nucleotide in accordance with item 66 wherein said M is hydrogen or a substitutable metal or a radioactive element.
79. The compound 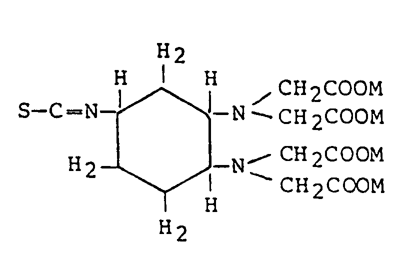 wherein M is hydrogen or a metal.
80. The compound 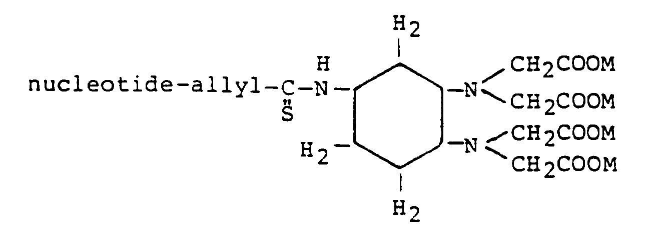 wherein M is hydrogen or a metal.
81. A compound in accordance with item 80 wherein said nucleotide is a deoxyribonucleotide.
82. A compound in accordance with item 80 wherein said nucleotide is a ribonucleotide.
83. The compound 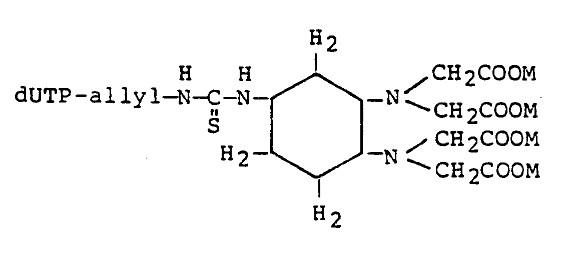 wherein M is hydrogen or a metal.
84. A nucleotide in accordance with items 1, 49 or 65 wherein the Sig chemical moiety comprises the chemical moiety 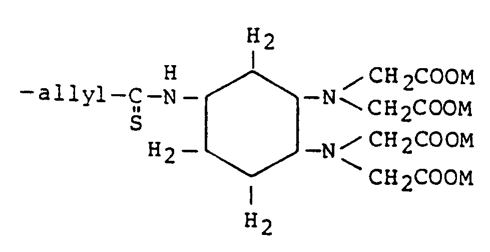
85. A polynucleotide containing one or more nucleotides in accordance with item 84.
86. A Polynucleotide in accordance with item 85 wherein said polynucleotide is a Single-stranded deoxyribonucleotide or a single-stranded ribonucleotide or a double-stranded DNA, RNA or DNA-RNA hybrid.
87. A nucleotide in accordance with item 1, wherein said Sig component comprises a radioactive component.
88. A single-stranded polynucleotide comprising one or more nucleotides in accordance with item 1, wherein said Sig component is radioactive.
89. A double-stranded polynucleotide comprising one or more nucleotides in accordance with item 1 and wherein said Sig component thereof is radioactive.
90. A nucleotide in accordance with item 49, wherein said Sig component comprises a radioactive moiety.
91. A polynucleotide comprising a ribonucleotide in accordance with item 49 wherein said Sig moiety is radioactively labeled.
92. A nucleotide in accordance with item 65, wherein said Sig moiety thereof is radioactively labeled.
93. A polynucleotide comprising one or more nucleotides in accordance with item 65 wherein said Sig moiety thereof is radioactively labeled.

Of special importance and significance in the practices of this invention is the utilization of self-signaling or self-indicating or self-detecting nucleic acids, particularly such nucleic acids which are capable of being incorporated in double-stranded DNA and the like. Such self-signaling or self-detecting nucleic acids can be created by covalently attaching to an allylamine substituent making up a modified nucleotide in accordance with this invention a molecule which will chelate specific ions, e.g. heavy metals, rare earths, etc. In general, the chelated ion can be detected either (a) by radioactive emission or (b) by using the ion to catalyze a chromogenic or fluorogenic reaction.

By way of example, a solution of 3,4-dinitro phenol is reduced to 3,4-diamino cyclohexane

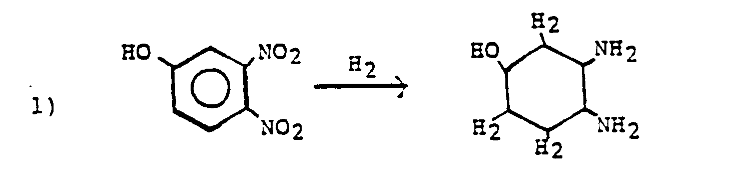

This material is then brominated 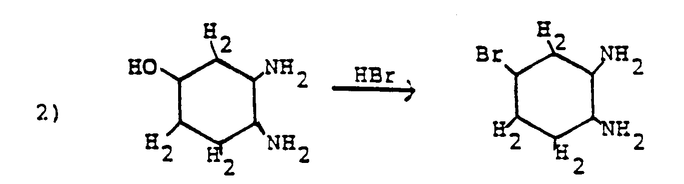
to form 3,4-diamino bromo cyclohexane (dABCH). This compound is reacted with halide (Cl, Br, I) substituted carboxymethyl compound to produce a tetra carboxymethyl derivative or dABCH (TCM-dABCH): 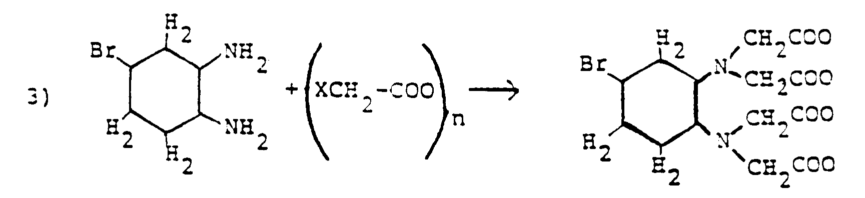
The bromine is substituted by an amino group using soluble ammonia: 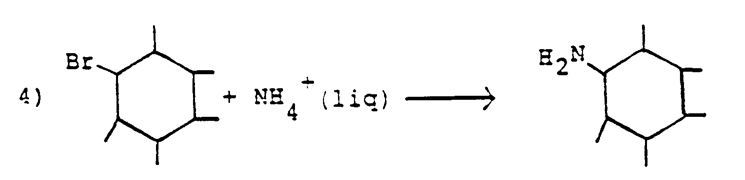
Then this compound is reacted with chloro thiophosgene to produce the isothiocyanate derivative of (TCM-dANCH). 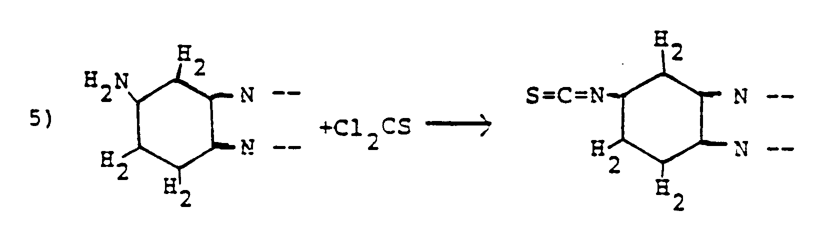
Finally, this compound is reacted with dUTP-allylamine derivative to produce modified dUTP. 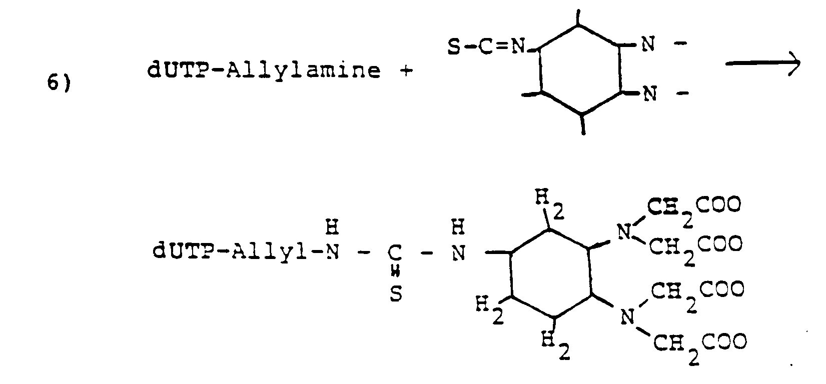
Cobalt or other heavy metal ions or other rare earth ions can be chelated to the compound after step 3 above. Or the nucleic acid can be substituted with this adduct and then the ion added. (Example, cobalt is added at pH 6 where the binding constant is 10⁻¹⁹M).

Cobalt can be assayed by radioactivity. It can also be detected by its ability to oxidize methylene blue to the leuco form in the presence of molecular oxygen. It can be used to oxidze soluble sulfhydro groups to disulfide bonds again in the presence of molecular oxygen.

This type of self-signaling molecule can be used to monitor any nucleic acid hybridization reaction. It is particularly important for detecting nucleic acids in gels (for example, sequencing gels).

With respect to its use in radioactivity, it can be used to tailor the isotope needed, i.e. if a weak or strong β or γ emitter is needed, that isotope can be chelated. Examples of isotopes that can be used are listed immediately hereinafter.

| | | |
|---|---|---|
| Antimony-124 | Iodine-125 | Scandium-44 |
| Antimony-125 | Iodine-131 | Scandium-46 |
| Arsenic-74 | Iodine-132 | Selenium-75 |
| | Iridium-192 | Silver-110m |
| Barium-133 | Iron-55 | Silver-111 |
| Barium-140 | Iron-59 | Sodium-22 |
| Beryllium-7 | | Strontium-85 |
| Bismuth-206 | Krypton-85 | Strontium-89 |
| Bismuth-207 | | Strontium-90 |
| | Lead-210 | Sulphur-35 |
| Cadmium-109 | Lutecium-177 | |
| Cadmium-115m | | Tantalum-182 |
| Calcium-45 | Manganese-54 | Technetium-99 |
| Carbon-14 | Mercury-197 | Tellurium-125m |
| Cerium-139 | Mercury-203 | Tellurium-132 |
| Cerium-141 | Molybdenum-99 | Terbium-160 |
| Cerium-144 | | Thallium-204 |
| Cesium-134 | Neodymium-147 | Thorium-228 |
| Cesium-137 | Neptunium-237 | Thorium-232 |
| Chlorine-36 | Nickel-63 | Thulium-170 |
| Chromium-51 | Niobium-95 | Tin-113 |
| Cobalt-56 | | Titanium-44 |
| Cobalt-57 | Osmium-185+191 | Tritium |
| Cobalt-58 | | Tungsten-185 |
| Cobalt-60 | Palladium-103 | |
| | | Vanadium-48 |
| Erbium-169 | Platinum-195m | Vanadium-49 |
| Europium-152 | Praseodymium-143 | |
| | Promethium-147 | Ytterbium-169 |
| Gadolinium-153 | Protactinium-233 | Yttrium-88 |
| Gold-195 | | Yttrium-90 |
| Gold-199 | Radium-226 | Yttrium-91 |
| | Rhenium-186 | |
| Hafnium-175 | Rubidium-86 | Zinc-65 |
| Hafnium-175+181 | Ruthenium-103 | Zirconium-95 |
| Hafnium-181 | Ruthenium-106 | |
| Hydrogen-3 see Tritium | | |

Another aspect of the practices of this invention which is particularly advantageous is to carry out the detection or hybridization in the liquid phase between the DNA sought to be detected and the DNA detecting probe. In this liquid phase system both the DNA molecule to be detected and the appropriate DNA detecting probe are not attached to any insoluble substrate or any insoluble chemical moiety. The advantages of the liquid phase detection system reside in the speed of hybridization or hybrid formation between the DNA to be detected and the appropriate DNA probe therefor. For example, in a solid-liquid system the time required to effect recognition and hybridization formation is about ten times greater than if it were carried out in a completely liquid system, i.e. both DNA to be detected and the detecting DNA are not attached to an insoluble moiety.

The probes prepared in accordance with the practices of this invention are adaptable for use in the detection of viruses and bacteria in fluids as indicated hereinabove. Where the fluids to be examined do not contain large amounts of protein, the viruses therein can be concentrated by absorption on hydroxyapatite and eluted in a small amount of phosphate buffer. When the fluid to be examined contains large amounts of protein, the viruses can be concentrated by high speed centrifugation.

If antibody were available, absorption on an affinity column and elution with acid would be preferable because it would be possible to process many probes in accordance with the practices of this invention at the same time. The bacteria to be examined are usually readily concentrated by centrifugation.

In accordance with the practices of this invention, the identification or characerization of the isolated particles, viruses and bacteria, would be hybridization of the characterizing or identifying DNA thereof with a specific single stranded DNA probe prepared in accordance with the practices of this invention. After hybridization, excess non-hybridized probe DNA would be digested with S₁ nuclease and exonuclease I from E. coli at a high salt content to suppress the nicking activity of the S₁ nuclease, see Vogt, Methods in Enzymology, Vol. 65, pages 248-255 (1980). This nuclease treatment would produce mononucleotides from the excess, non-hybridized single-stranded DNA probe but would leave the double-stranded, hybridised DNA intact. This would then be absorbed at a high salt content on a Dowex anion exchanger (the nucleotides and the small amount of oligonucleotides will not bind to the resin in high salt concentration). The resulting hybridized DNA would then be identified or characterized by various procedures applicable to the practices of this invention.

The special nucleotides of this invention include a phosphoric acid P moiety, a sugar or monosaccharide S moiety, a base B moiety, a purine or a pyrimidine and a signalling chemical moiety Sig covalently attached thereto, either to the P, S or B moiety. Following are structural formulas of various base B moieties and nucleotides which are modified in accordance with the practices of this invention. 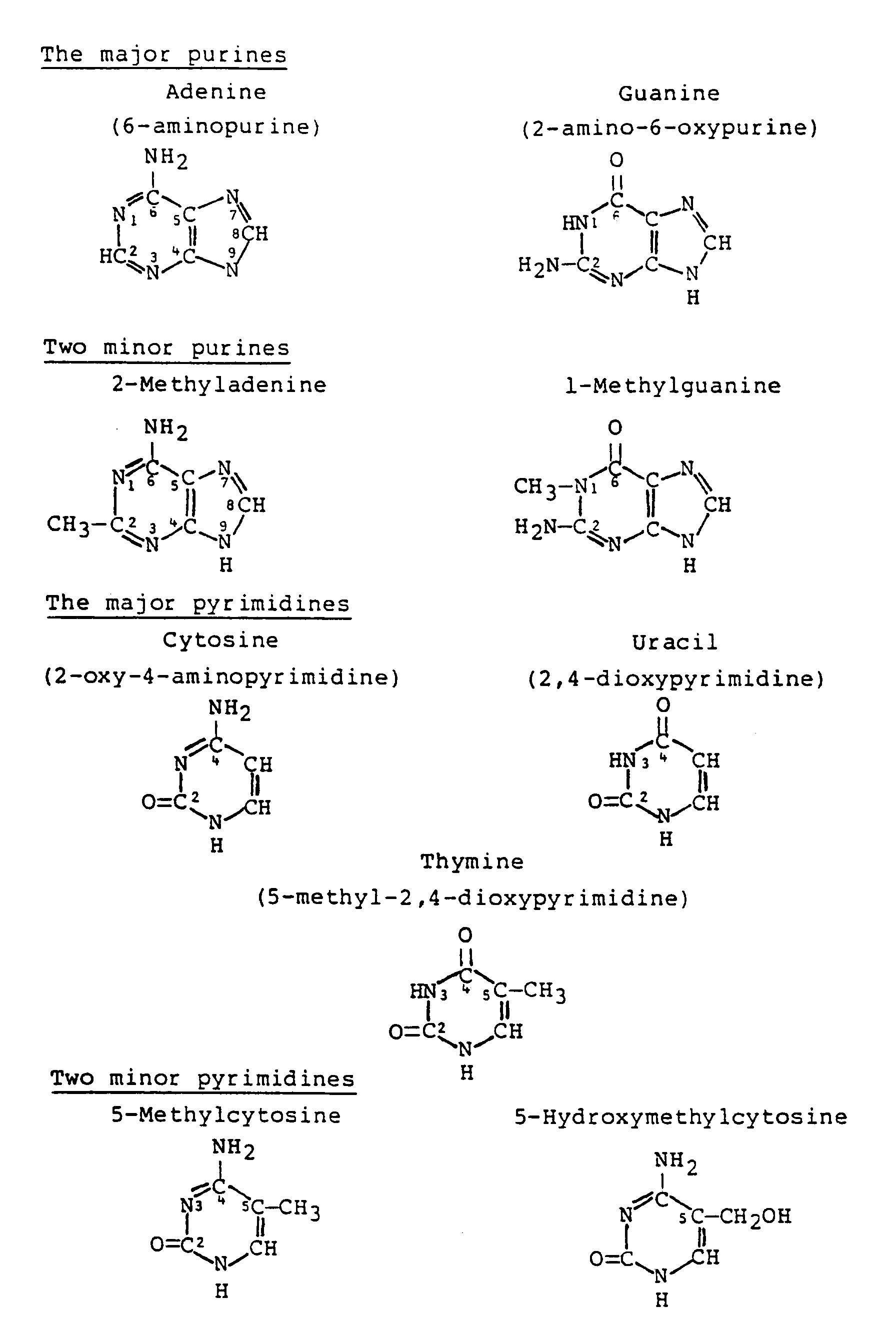 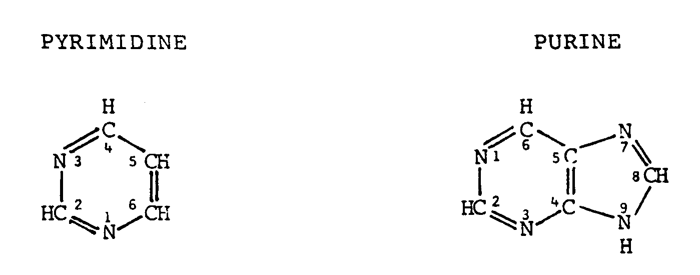
The major ribonucleotides and deoxyribonucleotides.

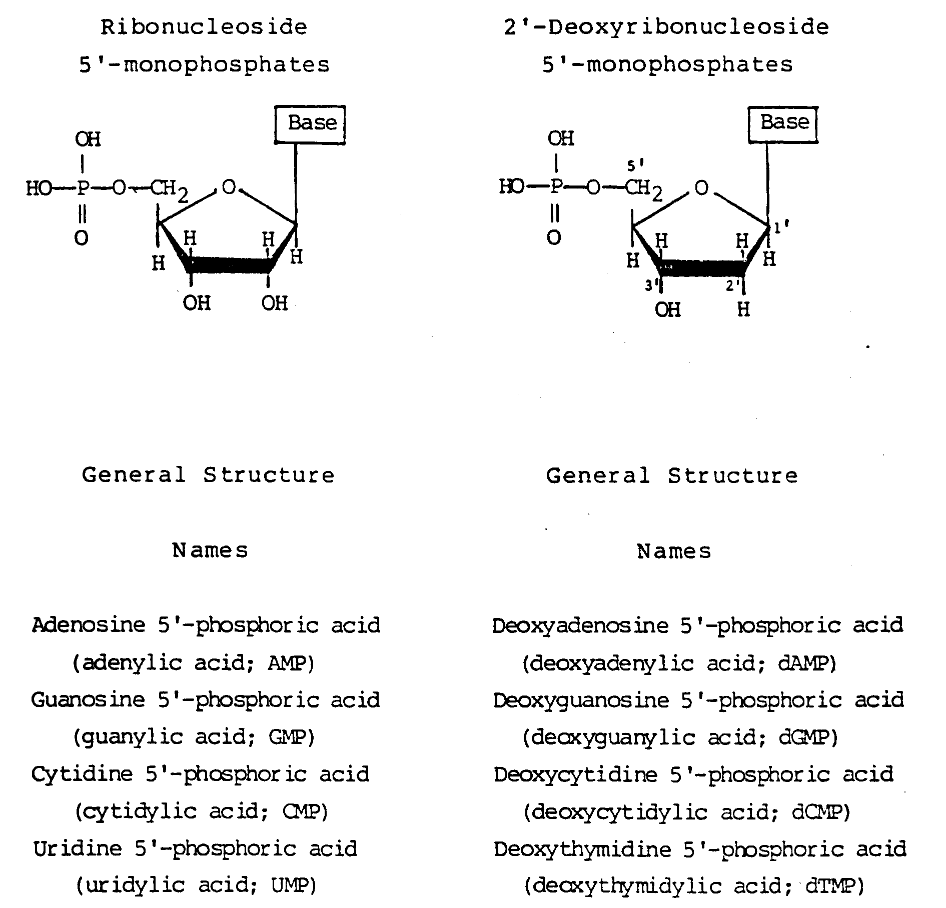

The special nucleotides in accordance with this invention, as indicated hereinabove, in addition to the P, S and B moieties, include a chemical moiety Sig coavalently attached to the P, S and/or B moieties. Of special interest in accordance with the practices of this invention would be those nucleotides having the general formula,

P - S - B - Sig

wherein P is the phosphoric acid moiety including mono-, di-, tri- or tetraphosphate, S the sugar monosaccharide moiety, B the base moiety, either a purine or a pyrimidine. The phosphoric acid moiety P is attached at the 3' and/or the 5' position of the S moiety when the nucleotide is a deoxyribonucleotide and at the 2', 3' and/or 5' position when the nucleotide is a ribonucleotide. The base B moiety is attached from the N1 position or the N9 position to the 1' position of the S moiety when the base moiety is a pyrimidine or a purine, respectively. The Sig moiety is covalently attached to the B moiety of the nucleotide and when so attached is capable of signalling itself or makes itself self-detecting or its presence known and desirably or preferably permits the incorporation of the resulting nucleotide P - S - B - Sig into or to form a double-stranded helical DNA or RNA or DNA-RNA hybrid and/or to be detectable thereon.

Another special nucleotide in accordance with this invention is characterized by the general formula: 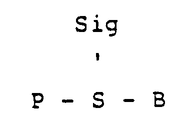
Such nucleotides in accordance with this invention would be characterized as ribonucleotides. The phosphoric acid moiety is attached at the 2', 3' and/or 5' position of the sugar S moiety and the base B being attached from the N1 position or the N9 position to the 1' position of the sugar S moiety when said base is a pyrimidine or a purine, respectively. The Sig chemical moiety is covalently attached to the sugar S moiety and said Sig chemical moiety where attached to said S moiety is capable of signalling itself or making itself self-detecting or its presence known and preferably permits the incorporation of the ribonucleotide into its corresponding double- stranded RNA or a DNA-RNA hybrid.

Such nucleotides 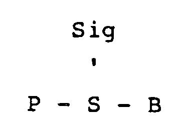
desirably have the Sig chemical moiety attached to the C2' position of the S moiety or the C3' position of the S moiety.

Still further, nucleotides in accordance with the practices of this invention include the nucleotides having the formula, 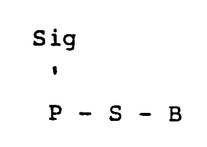
wherein P is the phosphoric acid moiety, S the sugar moiety and B the base moiety. In these special nucleotides the P moiety is attached to the 3' and/or the 5' position of the S moiety when the nucleotide is deoxyribonucleotide and at the 2', 3' and/or 5' position when the nucleotide is a ribonucleotide. The base B is either a purine or a pyrimidine and the B moiety is attached from the N1 or the N9 position to the 1' position of the sugar moiety when said B moiety is a pyrimidine or a purine, respectively. The Sig chemical moiety is covalently attached to the phosphoric acid P moiety via the chemical linkage 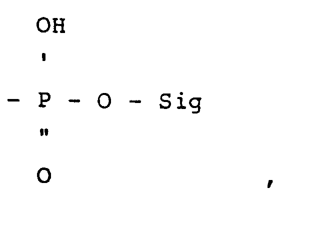
said Sig, when attached to said P moiety being capable of signalling itself or making itself self-detecting or its presence known and desirably the nucleotide is capable of being incorporated into a double-stranded polynucleotide, such as DNA, RNA or DNA-RNA hybrid and when so incorporated therein is still self- detecting.

It is pointed out that the special nucleotides in accordance with the practices of this invention described or defined hereinabove by the general formula P - S - B - Sig, also include nucleotides wherein the Sig chemical moiety is covalently attached to the B moiety at the N⁶ or 6- amino group position when the B moiety is adenine or the N² or 2-amino group position when the B moiety is guanine or the N⁴ or 4-amino group position when the B moiety is cytosine. The resulting nucleotides containing the Sig moiety attached thereto are capable of signalling themselves or making themselves self-detecting or their presence known and being detectable is a double-stranded or DNA, RNA or DNA-RNA hybrid.

By way of summary, as indicated hereinabove with respect to the make-up of the various special nucleotides in accordance with this invention, the special nucleotides can be described as comprising a phosphoric acid moiety P, a sugar moiety S and a base moiety B, a purine or pyrimidine, which combination of P-S-B is well known with respect to and defines nucleotides, both deoxyribonucleotides and ribonucleotides. The nucleotides are then modified in accordance with the practices of this invention by having covalently attached thereto, to the P moiety and/or the S moiety and/or the B moiety, a chemical moiety Sig. The chemical moiety Sig so attached to the nucleotide P-S-B is capable of rendering or making the resulting nucleotide, now comprising P-S-B with the Sig moiety being attached to one or more of the other moieties, self-detecting or signalling itself or capable of making its presence known per se, when incorporated into a polynucleotide, especially a double-stranded polynucleotide, such as a double-stranded DNA, a double-stranded RNA or a double-stranded. DNA-RNA hybrid. The Sig moiety desirably should not interfere with the capability of the nucleotide to form a double-stranded polynucleotide containing the special Sig-containing nucleotide in accordance with this invention and, when so incorporated therein, the Sig-containing nucleotide is capable of detection, localization or observation.

The Sig component of the nucleotides in accordance with this invention and the nucleotides and polynucleotides incorporating the nucleotides of this invention containing the Sig component are equivalent to and useful for the same purposes as the nucleotides described in EP-A2-0 063 879.

More specifically, the chemical moiety A described in EP-A2-0 063 879 is functionally the equivalent of the Sig component or chemical moiety of the special nucleotides of this invention. Accordingly, the Sig component or chemical moiety of nucleotides of this invention can be directly covalently attached to the P, S or B moieties or attached thereto via a chemical linkage or linkage arm as described in EP-A2-0 063 879 as indicated by the dotted line connecting B and A of the nucleotides of EP-A2-0 063 879 The various linker arms or linkages identified in EP-A2-0 063 879 are applicable to and useful in the preparation of the special nucleotides of this invention.

A particularly important and useful aspect of the special nucleotides of this invention is the use of such nucleotides in the preparation of DNA or RNA probes. Such probes would contain a nucleotide sequence substantially matching the DNA or RNA sequence of genetic material to be located and/or identified. The probe would contain one or more of the special nucleotides of this invention. A probe having a desired nucleotide sequence, such as a single- stranded polynucleotide, either DNA or RNA probe, would then be brought into contact with DNA or RNA genetic material to be identified. Upon the localization of the probe and the formation of a double-stranded polynucleotide containing the probe and the matching DNA or RNA material to be identified, the resulting formed double-stranded DNA or RNA-containing material would then be observable and identified. A probe in accordance with this invention may contain substantially any number of nucleotide units, from about 5 nucleotides up to about 500 or more, as may be required. It would appear that 12 matching, preferably consecutive, nucleotide units would be sufficient to effect an identification of most of the DNA or RNA material to be investigated or identified, if the 12 nucleotide sequence of the probe matches a corresponding cooperative sequence in the DNA or RNA material being investigated or to be identified. As indicated, such probes may contain one or more of the special Sig-containing nucleotides in accordance with this invention, preferably at least about one special nucleotide per 5-10 of the nucleotides in the probe.

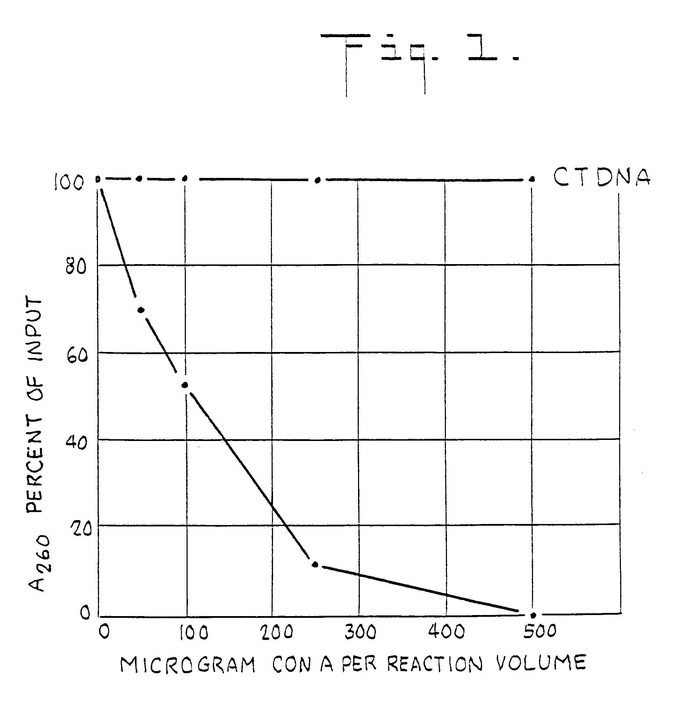

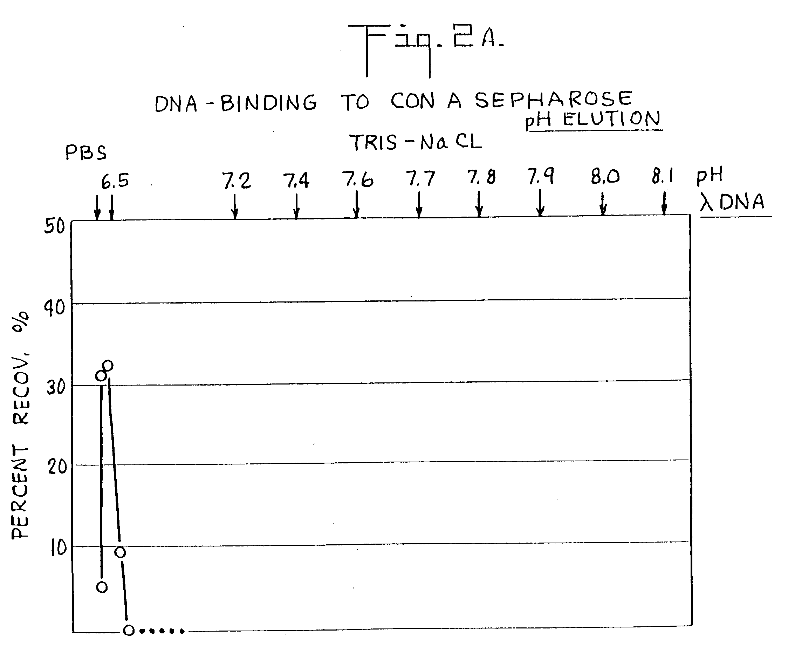

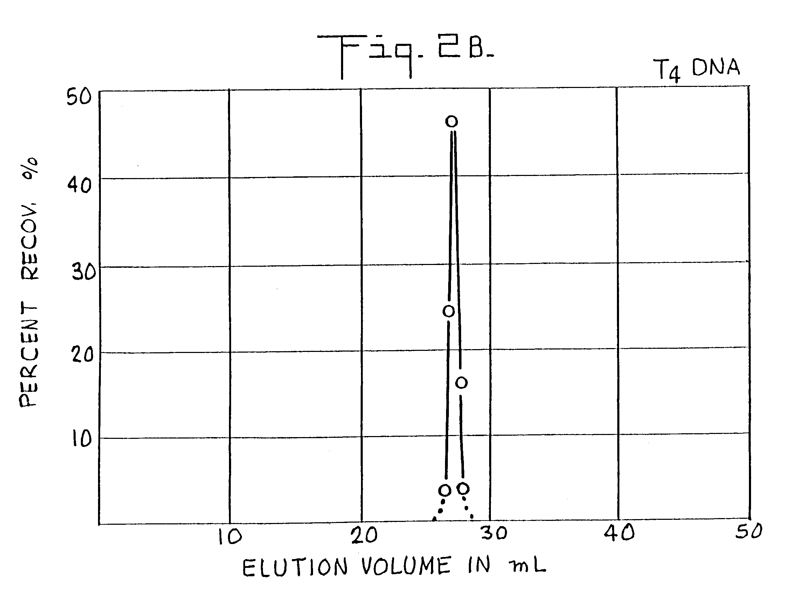

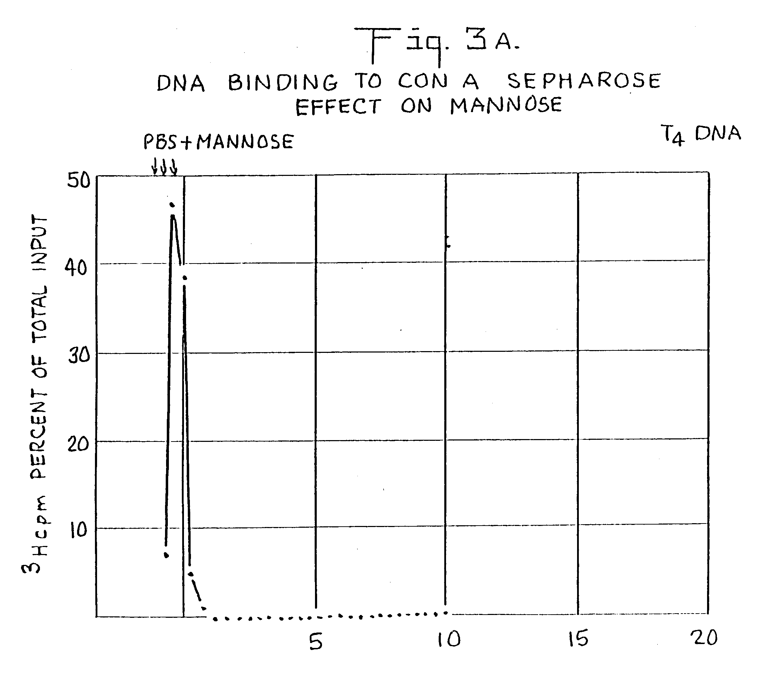

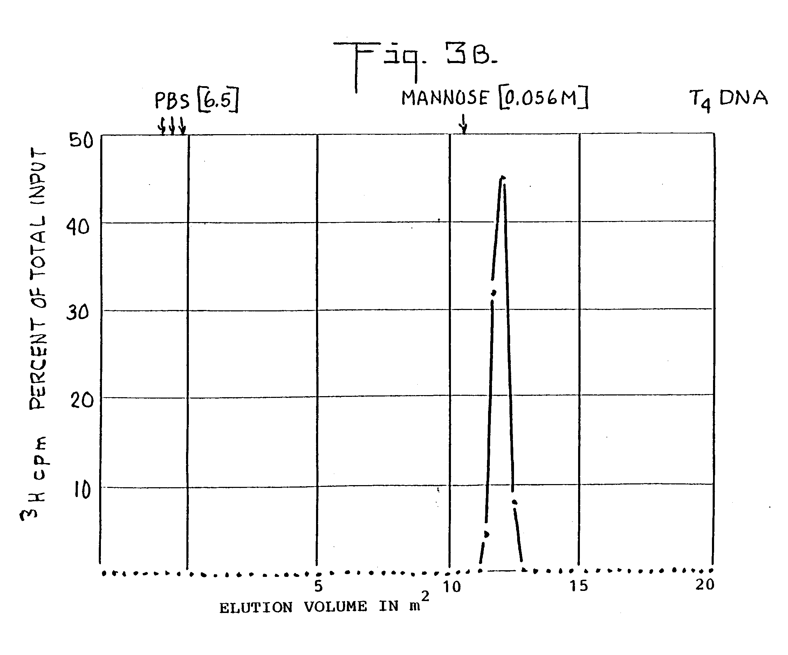

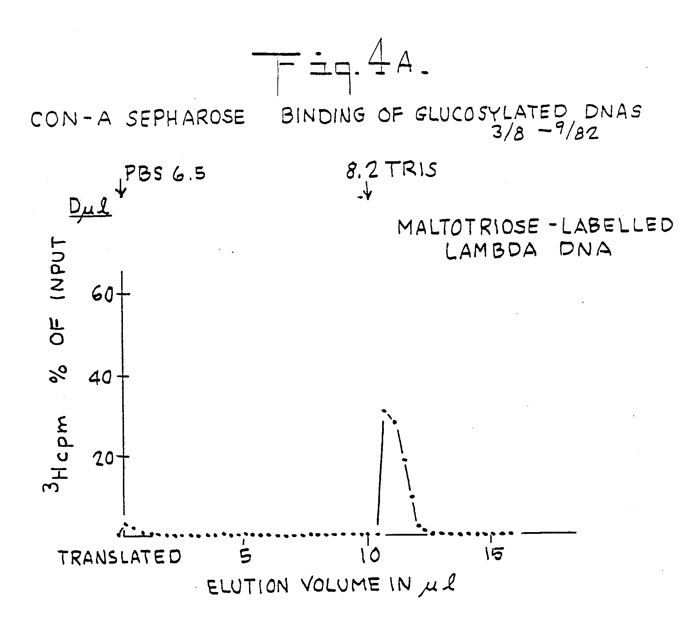

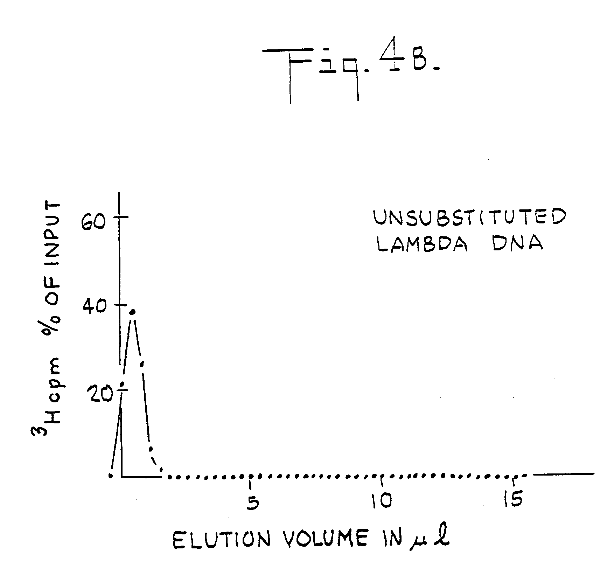

## Claims

1. A nucleotide to which is covalently attached a moiety that is capable of chelating an ion.

2. The nucleotide according to claim 1 selected from the group consisting of:
(i) a nucleotide having the formula
P-S-B-Sig
wherein P is a phosphate moiety, S is a sugar moiety, B is a pyrimidine, purine or 7-deazapurine moiety, Sig comprises a moiety capable of chelating a detectable ion, P being attached at the 3' or the 5' position of S when the nucleotide is a deoxyribonucleotide and at the 2', 3' or 5' position when the nucleotide is a ribonucleotide, B being attached to the 1' position of S from the N¹ position when B is a pyrimidine or the N⁹ position when B is a purine or a 7-deazapurine, and Sig is directly or indirectly attached to B at a position other than the C⁵ position when B is a pyrimidine, at a position other than the C⁸ position when B is a purine and at a position other than the C⁷ position when B is a 7-deazapurine;
(ii) a ribonucleotide having the formula, 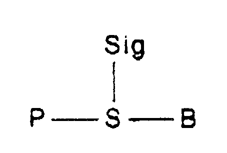 wherein P is a phosphate moiety, S is a sugar moiety, B is a pyrimidine purine or 7-deazapurine moiety, and Sig comprises a moiety capable of chelating a detectable ion, P being attached at the 2', 3' or 5' position of S, B being attached to the 1' position of S from the N¹ position when B is a pyrimidine or the N⁹ position when B is a purine or 7-deazapurine; and Sig is directly or indirectly attached to S; and
(iii) a nucleotide having the formula, 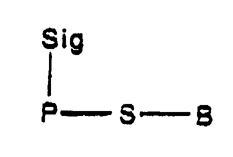 wherein P is a phosphate moiety, S is a sugar moiety, B is a pyrimidine, purine or 7-deazapurine moiety, Sig comprises a moiety capable of chelating a detectable ion, P being attached to the 3' or the 5' position of S when said nucleotide is a deoxyribonucleotide and at the 2', 3' or 5' position when said nucleotide is a ribonucleotide, B being attached to the 1' position of S from the N¹ position when B is a pyrimidine or the N⁹ position when B is a purine, and wherein Sig is directly or indirectly attached to P.

3. The nucleotide according to claim 2, wherein the moiety that is capable of chelating ions comprises two nitrogens and four carboxyl-containing groups, wherein two of said carboxyl-containing groups are attached to each of said nitrogens.

4. The nucleotide according to claim 3, wherein the moiety that is capable of chelating ions is a carboxy-substituted diamine.

5. The nucleotide according to claim 4, wherein the moiety that is capable of chelating ions has the structural formula: 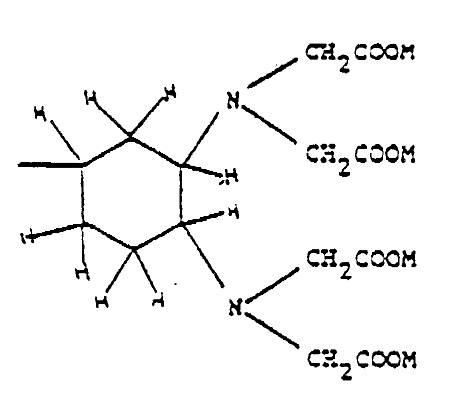 wherein M is hydrogen or a suitable cation.

6. The nucleotide according to claim 5, wherein the moiety that is capable of chelating ions has the structural formula: 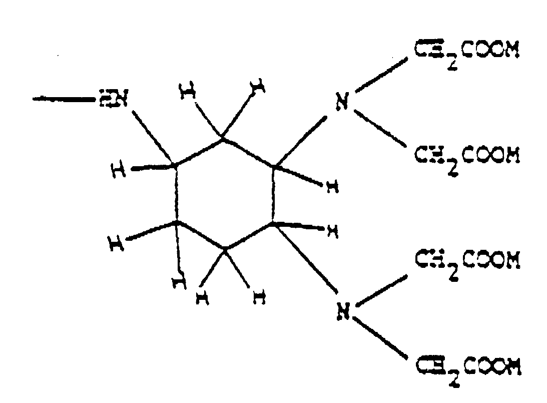 wherein M is hydrogen or a suitable cation.

7. The nucleotide according to claim 6, wherein the moiety that is capable of chelating ions has the structural formula: 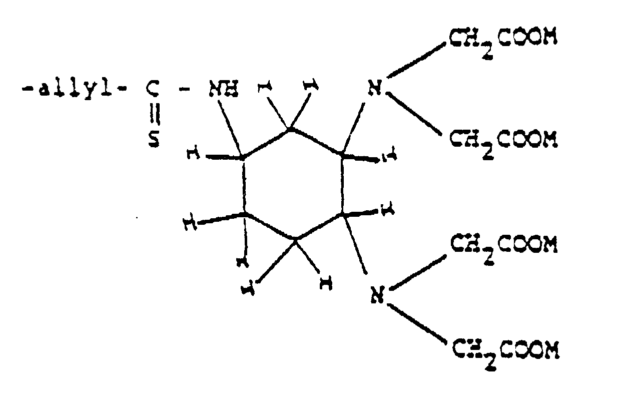 wherein M is hydrogen or a suitable cation.

8. The nucleotide according to claim 1, wherein the ion is selected from the group consisting of an ion of a radioactive metal, an ion of a metal capable of catalyzing a chromogenic or fluorogenic reaction and an ion of a metal capable of fluorescing or chemiluminescing alone or when in combination with another compound.

9. The nucleotide according to claim 1, wherein the moiety that is capable of chelating ions is covalently attached to the base, sugar or phosphorus-containing moiety through a linkage group, the linkage group including an olefinic bond at the α-position relative to the point of attachment to the nucleotide and/or including a -CH₂NH-moiety not interfering substantially with the characteristic ability of the moiety to chelate ions.

10. The nucleotide according to claim 1 further comprising a heavy metal or rare earth ion, the ion being chelated by the moiety that is capable of chelating ions.

11. The nucleotide according to claim 9, wherein the linkage group comprises an allylamine group.

12. The compound: 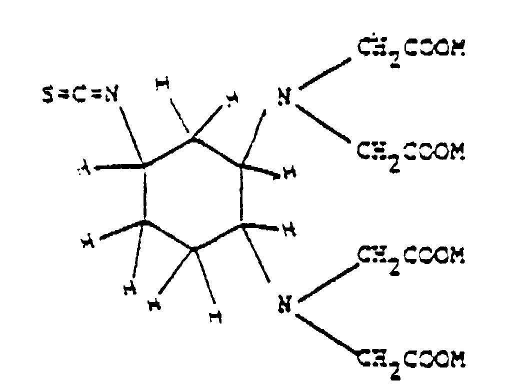 wherein M is hydrogen or a suitable cation.

13. The compound: 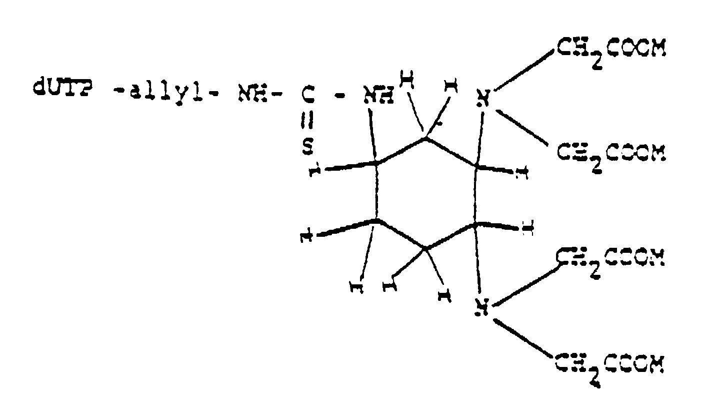 wherein M is hydrogen or a suitable cation.

14. An oligo- or polynucleotide characterized by at least one moiety that is capable of chelating ions, the moiety being attached to at least one nucleotide of the oligo- or polynucleotide.

15. The oligo- or polynucleotide according to claim 14, wherein said moiety that is capable of chelating ions comprises two nitrogens and four carboxyl-containing groups, wherein two of said four carboxyl-containing groups are attached to each of said nitrogens.

16. The nucleotide according to claim 10, wherein the ion is selected from the group consisting of an ion of a radioactive metal, an ion of a metal capable of catalyzing a chromogenic or fluorogenic reaction and an ion of a metal capable of fluorescing or chemiluminescing alone or when in combination with another compound.

17. The nucleotide according to claim 16, wherein the radioactive metal is an isotope of bismuth, nickel, tin, technetium, mercury or cobalt.

18. The nucleotide according to claim 16, wherein the catalyzing metal is cobalt.

19. The nucleotide according to claim 1, wherein the phosphorus-containing moiety is a mono-,di or tri-phosphate and the chemical moiety is covalently attached to the phosphorus-containing moiety through a phosphate oxygen.

20. The nucleotide according to claim 1 wherein said ion comprises a detectable ion.

## Patentansprüche

1. Nucleotid, an das kovalent eine Einheit gebunden ist, die mit einem Ion ein Chelat bilden kann.

2. Nucleotid nach Anspruch 1, das
(i) ein Nucleotid mit der Formel
P-S-B-Sig
ist, wobei P eine Phosphateinheit, S eine Zuckereinheit, B eine Pyrimidin-, Purin- oder 7-Deazapurineinheit ist, Sig eine Einheit umfaßt, die mit einem nachweisbaren Ion ein Chelat bilden kann, P an die 3'- oder 5'-Position von S gebunden ist, wenn das Nucleotid ein Desoxyribonucleotid ist und an die 2'-, 3'- oder 5'-Position, wenn das Nucleotid ein Ribonucleotid ist, B über die N¹-Position an die 1'-Position von S gebunden ist, wenn B ein Pyrimidin ist und über die N⁹-Position, wenn B ein Purin oder ein 7-Deazapurin ist, und Sig direkt oder indirekt an B an eine Position gebunden ist, die nicht die C⁵-Position ist, wenn B ein Pyrimidin ist, die nicht die C⁸-Position ist, wenn B ein Purin ist, und die nicht die C⁷-Position ist, wenn B ein 7-Deazapurin ist;
(ii) ein Ribonucleotid mit der Formel 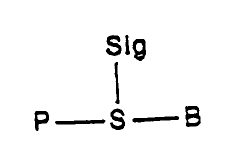 ist, wobei P eine Phosphateinheit, S eine Zuckereinheit, B eine Pyrimidin-, Purin- oder 7-Deazapurineinheit ist und Sig eine Einheit umfaßt, die mit einem nachweisbaren Ion ein Chelat bilden kann, P an die 2'-, 3'- oder 5'-Position von S gebunden ist, B über die N¹-Position an die 1'-Position von S gebunden ist, wenn B ein Pyrimidin ist, oder über die N⁹-Position, wenn B ein Purin oder 7-Deazapurin ist; und Sig direkt oder indirekt an S gebunden ist; oder
(iii) ein Nucleotid mit der Formel 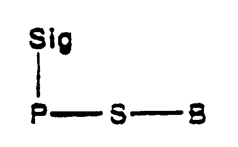 ist, wobei P eine Phosphateinheit, S eine Zuckereinheit, B eine Pyrimidin-, Purin- oder 7-Deazapurineinheit ist, Sig eine Einheit umfaßt, die mit einem nachweisbaren Ion ein Chelat bilden kann, P an die 3'- oder die 5'-Position von S gebunden ist, wenn das Nucleotid ein Desoxyribonucleotid ist, und an die 2'-, 3'- oder 5'-Position, wenn das Nucleotid ein Ribonucleotid ist, B über die N¹-Position an die 1'-Position von S gebunden ist, wenn B ein Pyrimidin ist, oder über die N⁹-Position, wenn B ein Purin ist, und Sig direkt oder indirekt an P gebunden ist.

3. Nucleotid nach Anspruch 2, wobei die Einheit, die mit Ionen Chelate bilden kann, zwei Stickstoffe und vier carboxylhaltige Gruppen umfaßt, und wobei zwei der carboxylhaltigen Gruppen an jeden der Stickstoffe gebunden sind.

4. Nucleotid nach Anspruch 3, wobei die Einheit, die mit Ionen Chelate bilden kann, ein carboxyl-substituiertes Diamin ist.

5. Nucleotid nach Anspruch 4, wobei die Einheit, die mit Ionen Chelate bilden kann, die folgende Strukturformel aufweist: 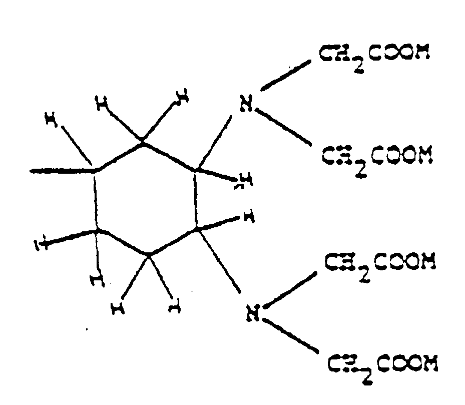 wobei M ein Wasserstoffatom oder ein geeignetes Kation ist.

6. Nucleotid nach Anspruch 5, wobei die Einheit, die mit Ionen Chelate bilden kann, die folgende Strukturformel aufweist: 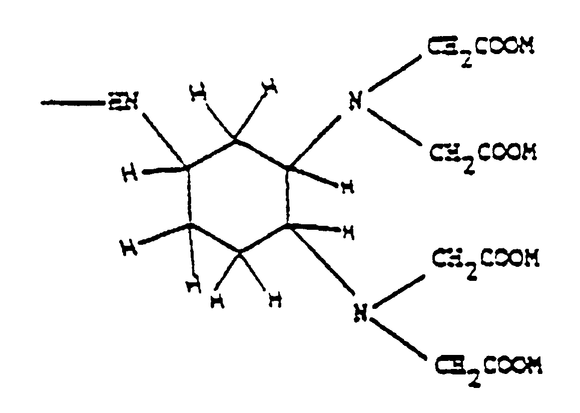 wobei M ein Wasserstoffatom oder ein geeignetes Kation ist.

7. Nucleotid nach Anspruch 6, wobei die Einheit, die mit Ionen Chelate bilden kann, die folgende Strukturformel aufweist 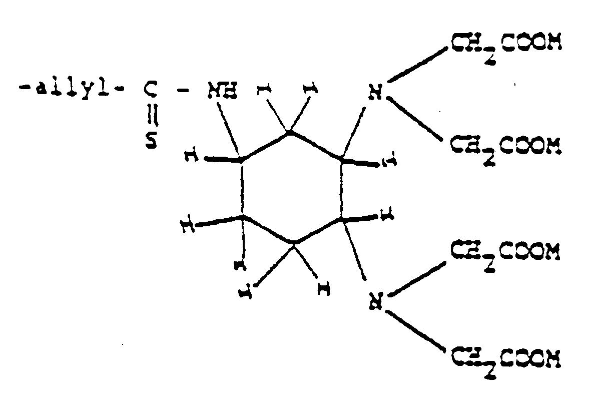 wobei M ein Wasserstoffatom oder ein geeignetes Kation ist.

8. Nucleotid nach Anspruch 1, wobei das Ion ein Ion eines radioaktiven Metalls, ein Ion eines Metalls, das eine chromogene oder fluorogene Reaktion katalysieren kann, oder ein Ion eines Metalls ist, das allein oder, wenn es in Kombination mit einem anderen Stoff vorliegt, fluoreszieren oder chemilumineszieren kann.

9. Nucleotid nach Anspruch 1, wobei die Einheit, die mit Ionen Chelate bilden kann, mit der Baseneinheit, Zuckereinheit oder Phosphor-enthaltenden Einheit kovalent über eine Brückengruppe gebunden ist, die Brückengruppe eine olefinische Bindung an der α-Position relativ zur Bindungsstelle an das Nucleotid und/oder eine -CH₂NH-Einheit enthält, die die kennzeichnende Fähigkeit der Einheit, mit Ionen Chelate zu bilden, nicht wesentlich stört.

10. Nucleotid nach Anspruch 1, das ferner ein Schwermetall- oder Seltene Erden-Ion umfaßt, wobei das Ion mit der Einheit ein Chelat bildet, die mit Ionen Chelate bilden kann.

11. Nucleotid nach Anspruch 9, wobei die Brückengruppe eine Allylaminogruppe umfaßt.

12. Verbindung 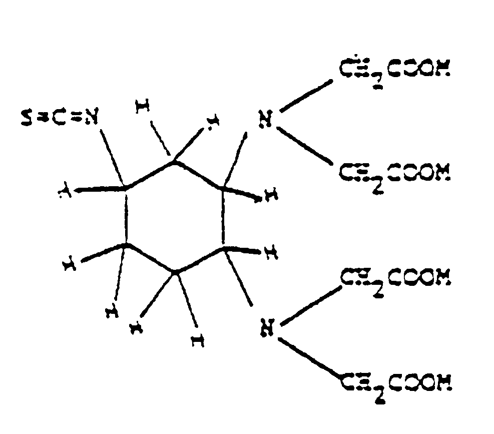 in der M ein Wasserstoffatom oder ein geeignetes Kation ist.

13. Verbindung 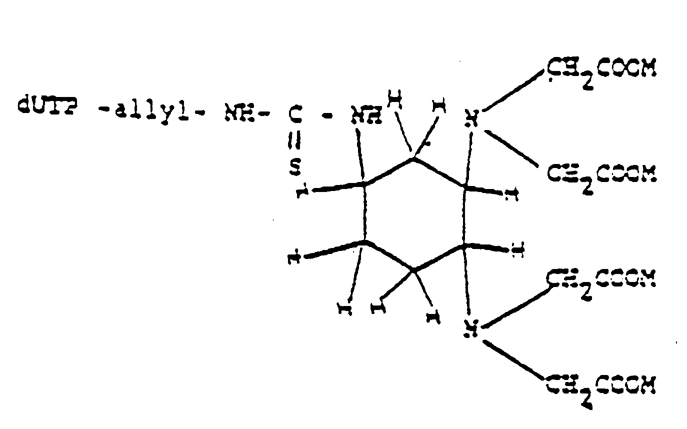 in der M ein Wasserstoffatom oder ein geeignetes Kation ist.

14. Oligo- oder Polynucleotid, gekennzeichnet durch mindestens eine Einheit, die mit Ionen Chelate bilden kann, wobei die Einheit an mindestens ein Nucleotid des Oligo- oder Polynucleotids gebunden ist.

15. Oligo- oder Polynucleotid nach Anspruch 14, wobei die Einheit, die mit Ionen Chelate bilden kann, zwei Stickstoffe und vier carboxylhaltige Gruppen umfaßt und zwei der vier carboxylhaltigen Gruppen an jeden der Stickstoffe gebunden sind.

16. Nucleotid nach Anspruch 10, wobei das Ion ein Ion eines radioaktiven Metalls, ein Ion eines Metalls, das eine chromogene oder fluorogene Reaktion katalysieren kann oder ein Ion eines Metalls ist, das allein oder, wenn es in Kombination mit einem anderen Stoff vorliegt, fluoreszieren oder chemilumineszieren kann.

17. Nucleotid nach Anspruch 16, wobei das radioaktive Metall ein Wismut-, Nickel, Zinn-, Technetium-, Quecksilber- oder Kobaltisotop ist.

18. Nucleotid nach Anspruch 16, wobei das katalysierende Metall Kobalt ist.

19. Nucleotid nach Anspruch 1, wobei die Phosphor-enthaltende Einheit ein Mono-, Di- oder Triphosphat ist und die chemische Einheit über einen Sauerstoff des Phosphats kovalent an die Phosphor-enthaltende Einheit gebunden ist.

20. Nucleotid nach Anspruch 1, wobei das Ion ein nachweisbares Ion umfaßt.

## Revendications

1. Un nucléotide auquel est fixé de façon covalente une fraction qui est susceptible de chelater un ion.

2. Le nucléotide selon la revendication 1, choisi dans le groupe constitué de :
(i) un nucléotide ayant la formule
P-S-B-Sig
dans laquelle P est une fraction phosphate, S est une fraction sucre, B est une fraction pyrimidine, purine ou 7-déazapurine, Sig comporte une fraction susceptible de chelater un ion détectable, P étant fixé sur la position 3' ou la position 5' de S lorsque le nucléotide est un désoxyribonucléotide et sur la position 2', 3' ou 5' lorsque le nucléotide est un ribonucléotide, B étant fixé sur la position 1' de S à partir de la position N¹ lorsque B est une pyrimidine ou sur la position N⁹ lorsque B est une purine ou une 7-déazapurine, et Sig est directement ou indirectement fixé à B sur une position autre que la position C⁵ lorsque B est une pyrimidine, sur une position autre que la position C⁸ lorsque B est une purine et sur une position autre que la position C⁷ lorsque B est une 7-déazapurine ;
(ii) un ribonucléotide ayant la formule, 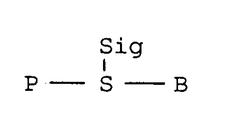 dans laquelle P est une fraction phosphate, S est une fraction sucre, B est une fraction pyrimidine, purine ou 7-déazapurine, et Sig comporte une fraction susceptible de chelater un ion détectable, P étant fixé sur les positions 2', 3' ou 5' de S, B étant fixé sur la position 1' de S à partir de la position N¹ lorsque B est une pyrimidine ou de la position N⁹ lorsque B est une purine ou une 7-déazapurine ; et Sig est directement ou indirectement fixé à S ; et
(iii) un nucléotide ayant la formule 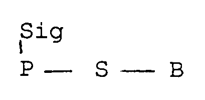 dans laquelle P est une fraction phosphate, S est une fraction sucre, B est une fraction pyrimidine, purine ou 7-déazapurine, Sig comporte une fraction susceptible de chelater un ion détectable, P étant fixé dans la position 3' ou dans la position 5' de S lorsque ledit nucléotide est un désoxyribonucléotide et dans la position 2', 3' ou 5' lorsque ledit nucléotide est un ribonucléotide, B étant fixé dans la position 1' de S à partir de la position N¹ lorsque B est une pyrimidine ou dans la position N⁹ lorsque B est une purine, et dans laquelle Sig est directement ou indirectement fixé à P.

3. Le nucléotide selon la revendication 2, dans lequel la partie qui est susceptible de chelater des ions comporte deux azotes et quatre groupes renfermant du carboxyle, deux desdits groupes renfermant du carboxyle étant fixés à chacun desdits azotes.

4. Le nucléotide selon la revendication 3, dans lequel la partie qui est susceptible de chelater des ions est une diamine à substitution carboxy.

5. Le nucléotide selon la revendication 4, dans lequel la partie qui est susceptible de chelater des ions présente la formule structurelle 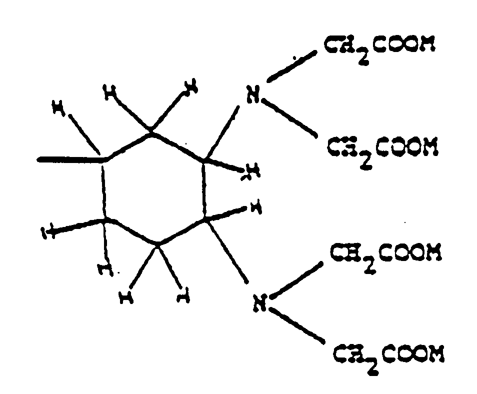 dans laquelle M est de l'hydrogène ou un cation approprié.

6. Le nucléotide selon la revendication 5, dans lequel la partie qui est susceptible de chelater des ions présente la formule structurelle : 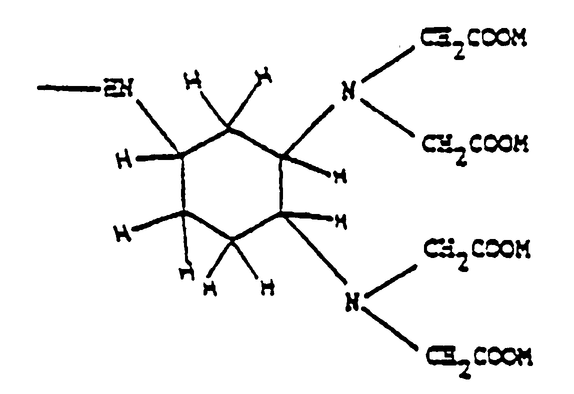 dans laquelle M est de l'hydrogène ou un cation approprié.

7. Le nucléotide selon la revendication 6, dans lequel la fraction qui est susceptible de chelater des ions présente la formule structurelle : 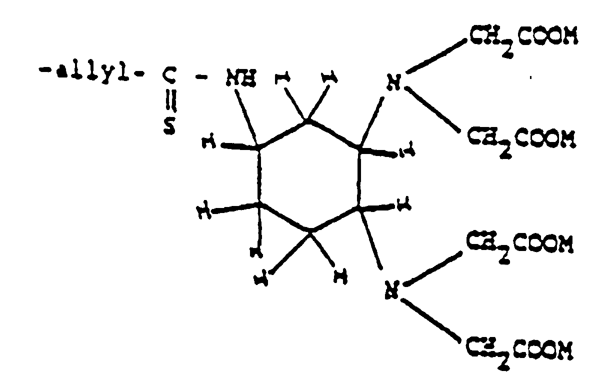 dans laquelle M est de l'hydrogène ou un cation approprié.

8. Le nucléotide selon la revendication 1, dans lequel l'ion est choisi dans le groupe constitué d'un ion de métal radioactif, d'un ion d'un métal susceptible de catalyser une réaction chromogène ou fluorogène et un ion d'un métal capable de produire de la fluorescence ou de la luminescence chimique seul ou lorsqu'il est combiné avec un autre composé.

9. Le nucléotide selon la revendication 1, dans lequel la fraction qui est susceptible de chelater des ions est fixée de façon covalente à la fraction de base, la fraction du sucre ou la fraction du phosphore par l'intermédiaire d'un groupe de liaison, le groupe de liaison comportant une liaison oléfinique en position α par rapport au point de fixation du nucléotide et/ou comportant une fraction -CH₂NH-n'interférant pas en pratique avec l'aptitude caractéristique de la fraction à chelater des ions.

10. Le nucléotide selon la revendication 1, comportant en outre un ion de métal lourd ou de métal de terres rares, cet ion étant chelaté par la fraction qui est susceptible de chelater des ions.

11. Le nucléotide selon la revendication 9, dans lequel le groupe de liaison comporte un groupe allylamine.

12. Le composé 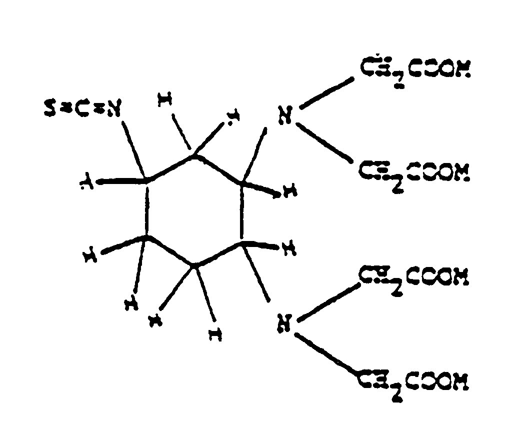 dans lequel M est de l'hydrogène ou un cation approprié.

13. Le composé : 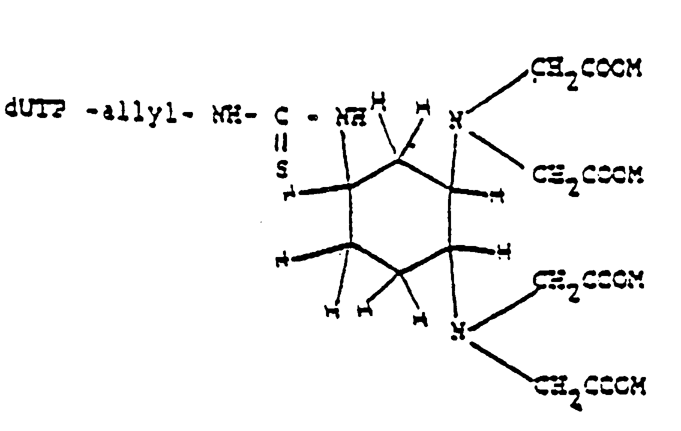 dans lequel M est de l'hydrogène ou un cation approprié.

14. Un oligo- ou polynucléotide caractérisé par au moins une fraction qui est susceptible de chelater des ions, la fraction étant fixée à au moins un nucléotide de l'oligonucléotide ou du polynucléotide.

15. L'oligo- ou polynucléotide selon la revendication 14, dans lequel ladite partie qui est susceptible de chelater des ions comporte deux azotes et quatre groupes renfermant du carboxyle, deux desdits groupes renfermant du carboxyle étant fixés à chacun desdits azotes.

16. Le nucléotide selon la revendication 10, dans lequel l'ion est choisi dans le groupe constitué d'un ion de métal radioactif, d'un ion d'un métal susceptible de catalyser une réaction chromogène ou fluorogène et d'un ion d'un métal susceptible de produire une fluorescence ou une luminescence chimique, seul ou lorsqu'il est combiné avec un autre composé.

17. Le nucléotide selon la revendication 16, dans lequel le métal radioactif est un isotope de bismuth, de nickel, d'étain, de technétium, de mercure ou de cobalt.

18. Le nucléotide selon la revendication 16, dans lequel le métal catalyseur est du cobalt.

19. Le nucléotide selon la revendication 1, dans lequel la fraction renfermant du phosphore est un mono-, di- ou tri-phosphate et la fraction chimique est fixée, de façon covalente, à la fraction renfermant du phosphore par l'intermédiaire d'un oxygène du phosphate.

20. Le nucléotide selon la revendication 1, dans lequel ledit ion comprend un ion détectable.
